# EUROPEAN PATENT SPECIFICATION

(11) **EP 4 267 058 B1**
(45) Date of publication and mention of the grant of the patent: **01.01.2025**
(21) Application number: 21827670.7
(22) Date of filing: 13.12.2021
(51) Int. Cl.: A61F 9/007

(54) **PROCESSOR-CONTROLLED PUMP IN IRRIGATION LINE OF PHACOEMULSIFICATION PROBE**
PROZESSORGESTEUERTE PUMPE IN EINER SPÜLLEITUNG EINER PHAKOEMULSIFIKATIONSSONDE
POMPE COMMANDÉE PAR PROCESSEUR DANS UNE LIGNE D'IRRIGATION DE SONDE DE PHACOÉMULSIFICATION

(30) Priority: 22.12.2020 US 202017130846
(43) Date of publication of application: 01.11.2023
(73) Proprietor: Johnson & Johnson Surgical Vision, Inc., Irvine, CA 92618 (US)
(72) Inventor: ALGAWI, Yehuda, 2066717 Yokneam (IL); GOVARI, Assaf, 2066717 Yokneam (IL); SITNITSKY, Ilya, 2066717 Yokneam (IL); AHARON, Eran, 2066717 Yokneam (IL)
(74) Representative: Carpmaels & Ransford LLP
(86) International application number: PCT/IB2021/061633
(87) International publication number: WO 2022/137006

(56) References cited:
- IE-A1- 920 003
- US-A1- 2013 131 692
- US-B2- 10 596 033

## Description

### FIELD OF THE INVENTION

The present invention relates generally to phacoemulsification, and specifically to irrigation in phacoemulsification systems.

### BACKGROUND OF THE INVENTION

A cataract is a clouding and hardening of the eye's natural lens, a structure which is positioned behind the cornea, iris and pupil. The lens is mostly made up of water and protein and as people age these proteins change and may begin to clump together obscuring portions of the lens. To correct this, a physician may recommend phacoemulsification cataract surgery. In the procedure, the surgeon makes a small incision in the sclera or cornea of the eye. Then a portion of the anterior surface of the lens capsule is removed to gain access to the cataract. The surgeon then uses a phacoemulsification probe, which has an ultrasonic handpiece with a needle. The tip of the needle vibrates at ultrasonic frequency to sculpt and emulsify the cataract while a pump aspirates particles and fluid from the eye through the tip. Aspirated fluids are replaced with irrigation of a balanced salt solution to maintain the anterior chamber of the eye. After removing the cataract with phacoemulsification, the softer outer lens cortex is removed with suction. An intraocular lens (IOL) is then introduced into the empty lens capsule restoring the patient's vision.

Various techniques of irrigation and/or aspiration with a phacoemulsification probe were proposed in the patent literature. For example, U.S. Patent 5,160,317 describes a method and apparatus for operating on the human eye that detects changes in load on the transducer and controls aspiration based on the load changes. A change from a lower load to a higher load indicates that harder tissue is being encountered and accordingly causes aspiration amount to increase. Conversely, a change from a higher load to a lower load indicates that aspiration amount should be quickly decreased since the tissue which is being encountered is softer.

As another example, U.S. Patent 10,052,227 describes a surgical apparatus configurable for removal of the cortex and the polishing of the capsule during cataract extraction surgery. In a configuration, a microprocessor-controlled pump and valve are used to calculate the specific fluid exchange such that the velocity and volume of the fluids extracted and introduced are relatively matched. It is envisioned that the microprocessor is equipped with sensors to detect the overall pressure in the ocular cavity and is configured to control the operation of the pump automatically.

U.S. Patent Application Publication 2019/0133822 describes a phacoemulsification system includes a console connected to a hand piece that has an ultrasonically vibrated cutting tip. The console generates an ultrasonic electrical signal for the cutting tip, provides irrigation fluid and is the source of aspiration force. The console also has a pressure sensor. The control system has automatic operation such that the aspiration force lowers when a first preset pressure limit is reached, and the aspiration force and ultrasonic vibration are turned off at a second preset pressure limit higher than the first. The control system also lowers the aspiration force initially when a change in pressure per unit of time exceeds a preset limit and then increases the aspiration flow if the pressure level drops below the limit. Since it is helpful with respect to pressure in the eye to have the irrigation and aspiration fluid flows balanced, in the case where there is an irrigation pump it should be controlled in the same way as the aspiration pump described above.

U.S. Patent Application Publication 2019/0099547 describes a system for detecting intraocular pressure events during phacoemulsification surgery having a surgical console, a handpiece having a proximal end being communicatively connected to an irrigation line and an aspiration line; a first sensor in communication with the aspiration line or the irrigation line for providing a first measurement value; and a second sensor in communication with the aspiration line for providing a second measurement value; and wherein at least one characteristic of the irrigation fluid or aspiration fluid in their respective lines is changed in accordance with the difference between the first measurement value and the second measurement value. A system for calibrating patient eye level and wound leakage; detecting occlusion or post occlusion surge; determining saline usage or remaining in a container; and detecting a fluid line abnormality using a first sensor and a second sensor in different configurations and differences in first and second measurement values. In one case, the surgical console may also be in communication with a pump, a gas reservoir, and a pressure regulator, each of which may be used for pressurized irrigation.

The disclosure of US10596033B2 provides a surgical instrument that includes a handpiece that includes a piezoelectric transducer. A hollow titanium needle having a substantially cylindrical portion and a free distal tip is attached to the handpiece by way of a threaded supported end structure. The piezoelectric transducer is driven by a circuit to periodically expand and contract at a highultrasound frequency that rings the hollow titanium needle with a high-ultrasonic frequency standing wave having a node of minimum amplitude residing in the substantially cylindrical portion between the supported end structure and the free distal tip, and to periodically expand and contract at an ultrasound frequency that rings the hollow titanium needle with an ultrasonic frequency standing wave, the circuit adapted to between the high-ultrasonic frequency and the ultrasonic frequency.

### SUMMARY OF THE INVENTION

The methods described herein are not encompassed by the wording of the claims but are considered as useful for understanding the invention.

A phacoemulsification system includes a phacoemulsification probe, an irrigation pump, an aspiration pump, circuitry, and a processor. The a phacoemulsification probe includes (i) a piezoelectric crystal configured to vibrate at a mechanical resonance frequency of the crystal, (ii) a needle, which is configured to be inserted into a lens capsule of an eye and to be vibrated by the piezoelectric crystal to emulsify a lens of the eye, (iii) an irrigation channel for receiving irrigation fluid from an irrigation line and flowing the irrigation fluid into the lens capsule, and (iv) an aspiration channel for removing material from the lens capsule and evacuating the removed material to an aspiration line. The irrigation pump is coupled with the irrigation line. The aspiration pump is coupled with the aspiration line. The circuitry is configured to measure electrical impedance of the piezoelectric crystal. The processor is configured to receive the measured electrical impedance, and to control an operation of the irrigation pump and the aspiration pump according to the measured electrical impedance.

In some embodiments, the processor is configured to control the operation by operating the irrigation pump and the aspiration pump simultaneously.

In some embodiments, the processor is configured to control the operation by operating a valve on at least one of the irrigation line and the aspiration line.

In other embodiments, the processor is configured to control the operation by operating a valve on at least one of an irrigation channel and an aspiration channel.

There is additionally provided
a method including inserting into an eye a phacoemulsification probe including a piezoelectric crystal, a needle, an irrigation channel, and an aspiration channel. The phacoemulsification needle is vibrated to emulsify a lens of the eye. Irrigation fluid is received from an irrigation line into an irrigation channel and the irrigation fluid is flew into a lens capsule of the eye. Material is removed from the lens capsule into an aspiration channel and the removed material is evacuated to an aspiration line.
measuring electrical impedance of the piezoelectric crystal. An operation of an irrigation pump and an aspiration pump, that are coupled with the irrigation line and the aspiration line, respectively, is controlled according to the measured electrical impedance.

The present invention will be more fully understood from the following detailed description of the embodiments thereof, taken together with the drawings in which:

### BRIEF DESCRIPTION OF THE DRAWINGS

Fig. 1 is a schematic, pictorial view, along with a side view, of a phacoemulsification system comprising a processor-controlled irrigation pump, in accordance with an embodiment of the present invention;
Fig. 2 is a block diagram, schematically describing a processor-controlled irrigation and aspiration sub-system of the phacoemulsification system of Fig. 1, in accordance with an embodiment of the present invention;
Fig. 3 is a flow chart schematically illustrating a method for adaptively operating irrigation and aspiration pumps of the irrigation and aspiration subsystem of Fig. 2;
Fig. 4 is a block diagram schematically describing processor-controlled irrigation and aspiration sub-systems of the phacoemulsification system of Fig. 1, in accordance with another embodiment of the present invention; and
Fig. 5 is a flow chart schematically illustrating a method for adaptively operating irrigation and aspiration pumps of the irrigation and aspiration subsystem of Fig. 4.

### DETAILED DESCRIPTION OF EMBODIMENTS

### OVERVIEW

During phacoemulsification of an eye lens, the eye is irrigated with a balanced salt solution to replace liquid and lens particles that are aspirated from the eye. The rate of irrigation should closely correspond to the rate of aspiration in order to prevent either under-pressure or over-pressure in the eye, either of which can cause trauma.

A phacoemulsification system may use a gravity feed for the irrigation, and the rate of irrigation may be controlled by an on/off valve and/or by adjusting the height of the irrigation source. However, such systems may produce traumatic effects, typically when there is a large change in a level of a vacuum in a pump-driven aspiration channel, for example due to blockage of the aspiration channel (e.g. an occlusion at the distal end of a needle), with either insufficient compensating change in the irrigation rate, or none at all.

Embodiments of the present invention that are described hereinafter incorporate a processor-controlled irrigation pump into the irrigation line and sensors which are configured to detect a change in pressure in the aspiration channel (e.g. a decrease or increase in pressure) before the change of pressure is present in the aspiration line. Once such pressure change is detected, the disclosed technique ensures fast response over the aspiration channel, such as immediate stop of aspiration, but at a same time ensures, using the irrigation pump, that no fallout occurs either, at the irrigation channel, such as over flow of fluid into the eye due to shutdown of aspiration. To this end, for example, a processor may command the irrigation pump to reduce rate of irrigation or halt the irrigation.

The processor-controlled irrigation pump may be incorporated with different embodiments of an aspiration sub-system, one of which includes an additional pump, such as a venturi pump, on the aspiration line. With such an incorporated pump and sensors, the disclosed technique provides an active processor-controlled irrigation and aspiration sub-system capable of suppressing the overall impact of a vacuum surge caused by an occlusion break by a large factor compared with existing solutions (e.g., to decrease time duration of the vacuum surge from 400 mSec to less than 200 mSec and amplitude of surge by factor of two, giving together a factor of 4 or more reduction in ratings of this traumatic event). Such fast response further requires fast response on the irrigation side.

In more detail, by using sensors at the back of the probe (e.g., handpiece) in a disclosed embodiment, the processor can initiate pump response in less than 4ms. By reversing the vacuum pump to decrease the vacuum level, and quickly increasing the irrigation flow rate momentarily, the vacuum surge time can be reduced to roughly 100-150ms, compared to 400ms in current systems, using the pumps alone.

In one embodiment, the irrigation pump responds to processor commands in a closed-loop control scheme, according to readings of a sensor located close to the eye, which, by sensing on the aspiration and/or irrigation channels in proximity to the eye, can indicate either an occurrence of an adverse event, such as a drop or an increase in intraocular pressure (IOP). As the sensor continuously monitors the IOP, the processor causes the pump to adaptively change/adjust based on the sensed IOP (e.g., making major or minor adjustments to flow rate, or even stopping flow, if required), so as to maintain the IOP within prespecified limits.

In some embodiments, the same processor that operates the irrigation pump also operates one or more aspiration pumps, and in some cases, simultaneously.

In some embodiments, in addition or alternatively to controlling the irrigation and aspiration pumps directly, the flow of fluids and materials through the system can be regulated by controlling valves. In an embodiment, the processor is configured to control an operation of the irrigation pump and/or the aspiration pump by opening and closing a valve on the irrigation and/or aspiration line. In another embodiment the processor controls an amount of partial opening of such valves.

As noted above, in some embodiments the processor receives readings in real time from a sensor, such as a pressure sensor or a flow sensor, that measures the irrigation fluid pressure (or flow rate) in the irrigation channel. The processor may receive readings from another sensor, such as a pressure sensor or a vacuum sensor, that measures the aspiration sub-pressure in the aspiration channel. In an embodiment, the measurements of pressure/flow (of irrigation) and pressure/vacuum (of aspiration) are performed at the probe, close to the irrigation outlet and aspiration inlet, respectively, so as to provide an early and accurate indication of the actual pressures experienced by the eye and provide a quick response time to a control loop of the irrigation and aspiration sub-system.

Using the pressure readings from the pressure and/or vacuum sensors, the processor adaptively operates the irrigation and aspiration pumps so as to maintain pressure and vacuum readings in the respective channels within prespecified limits.

In particular, as noted above, in the event of an occlusion occurring in the aspiration line, the disclosed technique provides a fast warning, and action to prevent a subsequent vacuum surge (e.g., indication within few milliseconds and suppression within several tens of mSec) to keep the IOP within prespecified limits, e.g., by early operating pumps simultaneously with both aspiration and irrigation lines.

In another embodiment, the irrigation pump responds to processor commands in a closed-loop control scheme, according to readings of sensing circuitry that senses an electrical impedance presented to the generator by the piezo-electric crystal that vibrates a needle comprising an aspiration channel. In case of an occlusion, the mechanical load of the needle on the piezo-electric crystal changes, and as a result the presented electrical impedance of the crystal changes, with that change being significant for a sensing signal having the resonance frequency or a frequency that falls within the resonance line shape.

As the amplitude changes when the needle is occluded, typically the impedance growing, this provides a timely warning of a possible vacuum surge to follow, once occlusion is released.

The impedance is measured using a circuitry that uses the drive frequency or a small amplitude signal with frequency falling in the line shape of the mechanical frequency (e.g., few % detuned from the resonance frequency). The aforementioned sensing circuitry measures the applied voltage and the current and determines from the voltage and current the changed impedance.

By incorporating and adaptively operating a pump on the irrigation line in a closed loop with distal sensors, the risk of trauma to the eye during a phacoemulsification procedure is reduced.

### SYSTEM DESCRIPTION

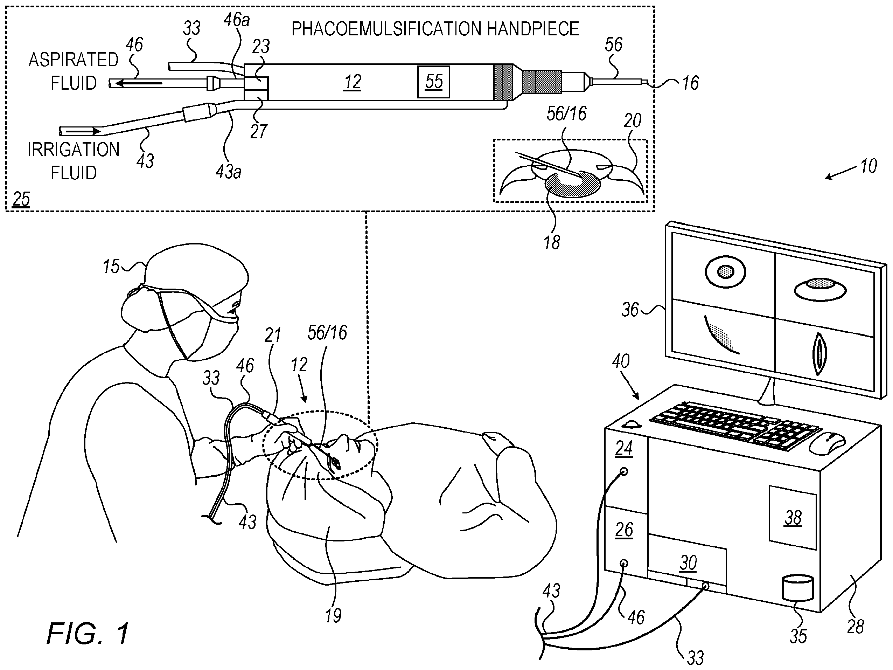

Fig. 1 is a schematic, pictorial view, along with a side view, of a phacoemulsification system 10 comprising a processor-controlled irrigation pump 24, in accordance with an embodiment of the present invention.

As seen in the pictorial view of phacoemulsification system 10, and in inset 25, phacoemulsification probe 12 (e.g., a handpiece 12) comprises a needle 16 surrounded by an irrigation sleeve 56. Needle 16 is hollow and its lumen is used as an aspiration channel.

Needle 16 is configured for insertion into a lens capsule 18 of an eye 20 of a patient 19 by a physician 15 to remove a cataract. The needle (and irrigation sleeve 56) are shown in inset 25 as a straight object. However, any suitable needle may be used with phacoemulsification probe 12, for example, a curved or bent tip needle commercially available from Johnson & Johnson Surgical Vision, Santa Ana, CA, USA.

In the shown example, probe 12 includes a sensor 23 coupled with irrigation channel 43a, and a sensor 27 coupled with aspiration channel 46a. Channels 43a and 46a are coupled respectively to irrigation line 43 and aspiration line 46. Sensors 23 and 27 may be any sensor known in the art, including, but not limited to a vacuum sensor or flow sensor. The sensor measurements (e.g. pressure, vacuum, and/or flow) are taken close to the proximal end of the handpiece where the irrigation outlet and the aspiration inlet are located, so as to provide processor 38 an accurate indication of the actual measurements occurring within an eye and provide a short response time to a control loop comprised in processor 38.

A vacuum sensor as discussed herein includes types of pressure sensors that are configured to provide sufficiently accurate measurements of low sub-atmospheric pressures within a typical sub-pressures range at which aspiration is applied (e.g., between 1 mmHg and 650 mmHg). In an embodiment, the same pressure sensor model is used to measure irrigation pressure and aspiration sub-pressure, using different sensor settings/calibrations.

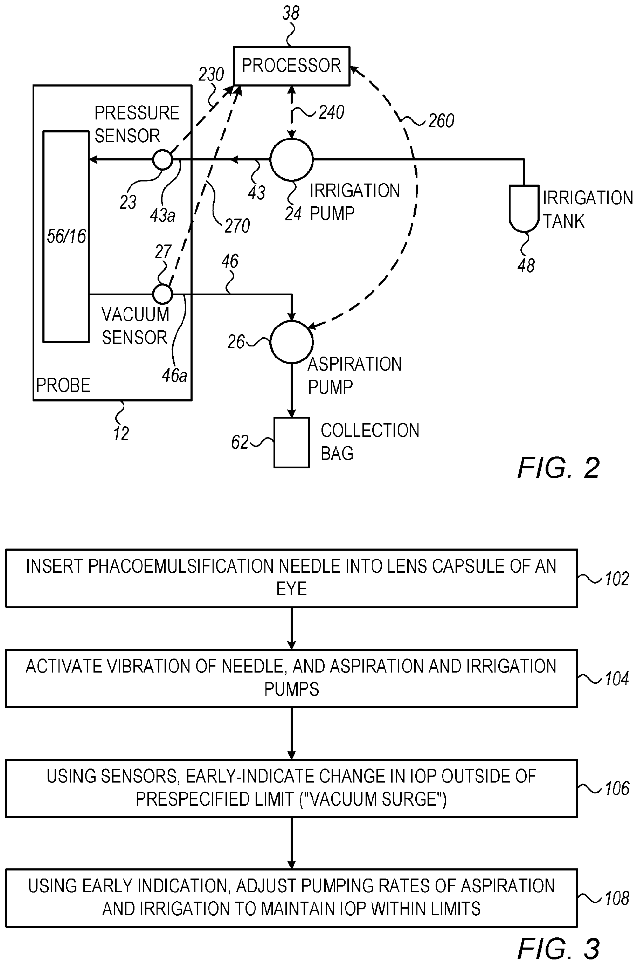

As shown, during the phacoemulsification procedure, processor-controlled pump 24 comprised in a console 28 pumps irrigation fluid from an irrigation reservoir or tank (shown in Fig. 2) via irrigation sleeve 56, to irrigate the eye. The fluid is pumped via irrigation tubing line 43 running from console 28 to probe 12. Irrigation pump 24 may be any pump known in the art, for example, a peristaltic pump, and using sensors (e.g. as indicated by sensors 23 and/or 27), processor 38 controls a pump rate of irrigation pump 24 to maintain intraocular pressure within prespecified limits.

Eye fluid and waste matter (e.g., emulsified parts of the cataract) are aspirated via hollow needle 16 to a collection receptacle (not shown) by a processor-controlled aspiration pump 26 also comprised in console 28 and using aspiration tubing line 46 running from probe 12 to console 28. In an embodiment, processor 38 controls an aspiration rate of aspiration pump 26 to maintain intraocular pressure (in case of sub-pressure indicated, for example, by sensor 27) within prespecified limits.

As further shown, phacoemulsification probe 12 includes a piezoelectric crystal 55, coupled to a horn (not shown), that drives needle 16 to vibrate in a resonant vibration mode that is used to break a cataract into small pieces during a phacoemulsification procedure. Console 28 comprises a piezoelectric drive module 30, coupled with the piezoelectric crystal, using electrical wiring running in cable 33. Drive module 30 is controlled by a processor 38 that uses the drive signal or a small-amplitude monitoring signal (e.g. at a detuned frequency) via cable 33 and enables an electrical impedance of crystal 55 to be monitored, to detect an occlusion and perform preemptive steps, such as adjusting the irrigation rate and/or adjusting the aspiration rate to prevent a subsequent vacuum surge.

Processor 38 further conveys processor-controlled driving signals via cable 33 to, for example, maintain needle 16 at maximal vibration amplitude. The drive module may be realized in hardware or software, for example, in a proportional-integral-derivative (PID) control architecture.

Processor 38 may receive user-based commands via a user interface 40, which may include setting a vibration mode and/or frequency of the piezoelectric crystal, and setting or adjusting an irrigation and/or aspiration rate of the irrigation pump 24 and aspiration pump 26. Processor 38 may receive user-based commands via a user interface 40, which may include needle 16 stroke amplitude settings and turning on irrigation and/or aspiration. In an embodiment, the physician uses a foot pedal (not shown) as a means of control. For example, pedal position one activates only irrigation, pedal position two activates both irrigation and aspiration, and pedal position three adds needle 16 vibration. Additionally, or alternatively, processor 38 may receive the user-based commands from controls located in a handle 21 of probe 12.

In an embodiment, user interface 40 and display 36 may be integrated into a touch screen graphical user interface.

Some or all of the functions of processor 38 may be combined in a single physical component or, alternatively, implemented using multiple physical components. These physical components may comprise hard-wired or programmable devices, or a combination of the two. In some embodiments, at least some of the functions of processor 38 may be carried out by suitable software stored in a memory 35 (as shown in Fig. 1). This software may be downloaded to a device in electronic form, over a network, for example. Alternatively, or additionally, the software may be stored in tangible, non-transitory computer-readable storage media, such as optical, magnetic, or electronic memory.

The system shown in Fig. 1 may include further elements, which are omitted for clarity of presentation. For example, physician 15 typically performs the procedure using a stereo microscope or magnifying glasses, neither of which are shown. Physician 15 may use other surgical tools in addition to probe 12, which are also not shown in order to maintain clarity and simplicity of presentation.

### PROCESSOR-CONTROLLED PUMP IN IRRIGATION LINE OF PHACOEMULSIFICATION PROBE

Fig. 2 is a block diagram schematically describing processor-controlled irrigation and aspiration sub-systems of phacoemulsification system 10 of Fig. 1, in accordance with an embodiment of the present invention.

As seen, processor 38 controls (240) irrigation pump 24 to pump balanced salt solution from an irrigation tank 48 to sleeve 56 (shown in Fig. 1), via irrigation line 43. At the same time, processor 38 controls (260) aspiration pump 26 to aspirate eye fluid into a collection bag 62, via aspiration line 46. Pumps 24 and 26 may include means to indicate actual pump performance (e.g., speed) to processor 38, for use by a feedback loop.

As further seen, sensor 23 and sensor 27 of probe 12 provide readings 230 (e.g. pressure, vacuum, or flow) of irrigation channel 43a, and readings 270 (e.g. pressure, vacuum, or flow) of aspiration channel 46a, respectively, to processor 38. The readings (230, 270) are provided in real time at a sufficiently high rate (e.g., 1 kHz) to allow a fast system response time (e.g., within several milliseconds). In the shown embodiment, sensors 23 and 27 are seen as located in the back of handpiece 12. In general, the sensors can be located in a case or module coupled with the handpiece (e.g., by including the sensors in a disposable case coupled to the aspiration and irrigation lines just proximally of the handle itself).

In response to readings (230, 270), processor 38 adjusts a rate of pumping of irrigation pump 24 and/or aspiration pump 26, to maintain readings (230, 270) within prespecified limits.

The example block diagram shown in Fig. 2 is highly simplified and was chosen purely for the sake of conceptual clarity. A typical system may include, for example, a complex aspiration apparatus, which may include two or more pumps, including a venturi pump. A phacoemulsification system, such as shown in of Fig. 1, additionally includes bypass protection, and may further include valves in irrigation/or aspiration lines.

Such and other details of an actual system are omitted for simplicity of presentation. In that regard, irrigation pump 24 represents an irrigation sub-system and aspiration pump 26 represents an aspiration sub-system, with their disclosed functionality described in essence above.

The term "sensor" includes any type of sensor that can provide indications to the processor running the control loop. For the aspiration channel, such a sensor may be a pressure sensor that is configured to provide sufficiently accurate measurements of low sub-atmospheric pressures that are within a typical range of sub-pressures at which aspiration is applied (e.g., between 1 mm Hg and 650 mm Hg). In an embodiment, sensors 23 and 27 comprise the same pressure sensor model, with different settings/calibrations to measure either irrigation pressure or aspiration sub-pressure. For the irrigation channel, such a sensor may be the aforementioned pressure sensor, or a fluid flow rate meter.

Fig. 3 is a flow chart schematically illustrating a method for adaptively operating irrigation pump (24) and aspiration (26) pump of the irrigation and aspiration subsystems of Fig. 2. The algorithm, according to the presented embodiment, carries out a process that begins with needle insertion step 102, during which physician 15 inserts phacoemulsification needle 16 of probe 12 into a lens capsule 18 of an eye 20.

At a phacoemulsification starting step 104, physician 15 vibrates needle 16 to break up a cataract and, at the same time, processor 38 activates the aforementioned irrigation pump 24 and aspiration pump 26.

Next, processor 38 receives readings (230, 270) from irrigation and aspiration channels 43 and 46, acquired by sensors 23 and 27, respectively, which early-indicate (e.g., within several mSec) a change in IOP outside a prespecified limit, at a drop in IOP indication receiving step 106. Using the early indication (e.g., at least one of readings (230, 270)), processor 38 calculates required changes in rates of pumps 24 and 26, and using the feedback loop that receives the early indication (e.g., feedback signals), processor 38 commands pumps 24 and 26 to adjust pump rates such that readings (230 and 270), indicative of the IOP, are maintained within prespecified limits, at pumps control step 108.

The example flow chart shown in Fig. 3 is chosen purely for the sake of conceptual clarity. For example, additional steps, such as controlling pumping systems 24 and 26, are omitted for simplicity and clarity of presentation. Furthermore, various irrigation/aspiration termination criteria, such as, for example, if a predefined number of control cycles fails to bring pressures to within limits, or if the failure is due to other problems, are omitted for simplicity of presentation.

In an embodiment, processor 38 checks if readings (230, 270) are within prespecified limits or ranges, e.g. above or below a threshold or between two set points. If they do, processor 38 continues applying the same irrigation and aspiration rates and the process returns to reading step 106. If they do not, processor 38 checks if the reading is outside the limits or range, e.g. is too high (indicating a risk of vacuum surge has been detected). If the reading is too high, processor 38 increases irrigation rate and/or lowers aspiration rate. The entire cycle described above (as well as the cycles described below), where readings are checked and corrective actions applied, typically requires no more than few milliseconds, thereby protecting the eye from any damage caused by irrigation and/or aspiration problems.

In another case, if reading 270 is too low (e.g., vacuum is insufficient), processor 38 may command pump 24 to lower irrigation flow. In yet another case, if reading 230 is too low (e.g., irrigation flow is insufficient), processor 38 may command pump 24 to increase irrigation flow.

### PROCESSOR-CONTROLLED PUMP IN IRRIGATION LINE IN RESPONSE TO DETECTED RISK OF VACCUMM SURGE

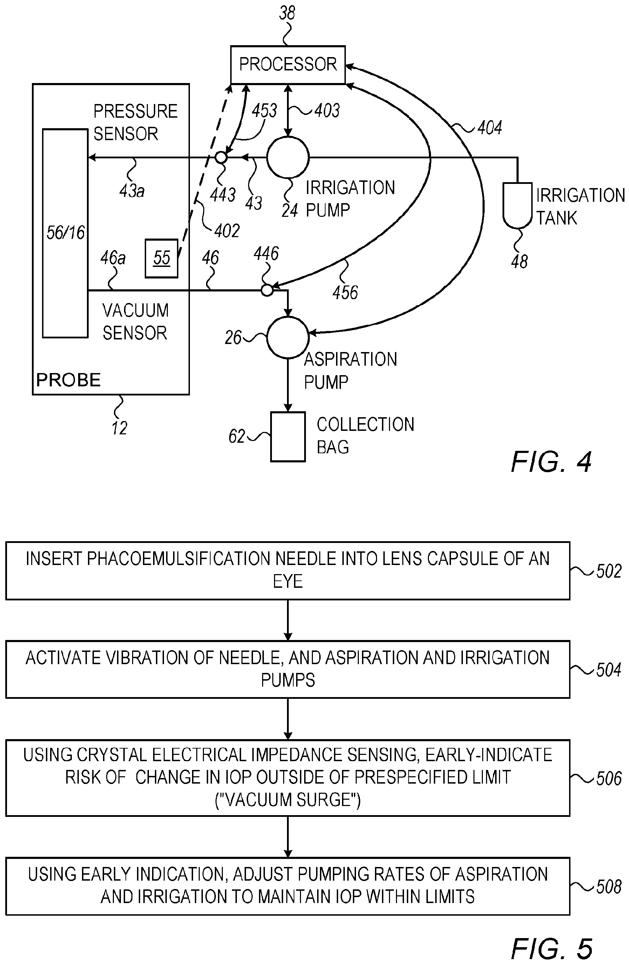

Fig. 4 is a block diagram schematically describing processor-controlled irrigation and aspiration sub-systems of phacoemulsification system 10 of Fig. 1, in accordance with another embodiment of the present invention.

As seen, processor 38 controls (403) irrigation pump 24 to pump balance salt solution from an irrigation tank 48 to sleeve 56, via irrigation line 43. At the same time, processor 38 controls (404) aspiration pump 26 to aspirate eye fluid into a collection bag 62, via aspiration line 46. Pumps 24 and 26 may include means to indicate actual pump performance (e.g., speed) to processor 38, for use by the feedback loop.

As further seen, electrical impedance of crystal 55 is sensed with readings (402), to detect occlusion and perform preemptive steps, such as adjusting irrigation rate and/or adjusting aspiration rate to prevent subsequent vacuum surge.

Readings (402) are provided in real time at a sufficiently high rate (e.g., 1 kHz) to allow a fast system response time (e.g., within several milliseconds).

In response to readings (402), processor 38 adjusts a rate of pumping of irrigation pump 24 and/or aspiration pump 26, until readings (402) return to prespecified limits or ranges, e.g. above or below a threshold or between two set points.

As noted above, in some embodiments, in addition or alternatively to controlling the irrigation and aspiration pumps directly, the flow of fluids and materials through the system can be regulated by controlling valves, such as controlling (453) valve 443 on the irrigation line and/or controlling (456) valve 446 on the aspiration line. In an embodiment, the processor is configured to control an operation of irrigation pump 24 and/or aspiration pump 26 by opening and closing valves 443 and/or 446. In another embodiment the processor controls an amount of partial opening of valves 443 and/or 446.

In other embodiments, the valves can be located inside handpiece 12, be coupled to irrigation and aspiration channels 43a and 46a, just proximally to handpiece 12, or be located elsewhere in the system, such as inside console 28.

Fig. 5 is a flow chart schematically illustrating a method for adaptively operating irrigation pump (24) and aspiration (26) pump of the irrigation and aspiration subsystems of Fig. 4.

The algorithm, according to the presented embodiment, carries out a process that begins with needle insertion step 502, during which physician 15 inserts phacoemulsification needle 16 of probe 12 into a lens capsule 18 of an eye 20.

At a phacoemulsification starting step 504, physician 15 vibrates needle 16 to break up a cataract and, at the same time, processor 38 activates the aforementioned irrigation pump 24 and aspiration pump 26.

Next, processor 38 receives pressure crystal's 55 electrical impedance readings (402) from crystal 55, which early-indicate (e.g., within several mSec) a risk of a change (e.g. drop) in IOP that is above or below a prespecified limit or outside a prespecified range, at a risk of IOP change indication receiving step 506. In an example, the IOP change may be a drop in IOP below a prespecified limit indicating a possible post occlusion surge risk. Using the early indication, processor 38 calculates required changes in rates of pumps 24 and 26, and, using the feedback loop that receives the early indication (e.g., feedback signals), processor 38 commands pumps 24 and 26 to adjust pump rates until that readings (402), return to prespecified limits, at pumps control step 508.

It will be appreciated that the embodiments described above are cited by way of example, and that the present invention is not limited to what has been particularly shown and described hereinabove. Rather, the scope of the present invention is defined by the appended claims, and includes both combinations and sub-combinations of the various features described hereinabove, as well as variations and modifications thereof which would occur to persons skilled in the art upon reading the foregoing description and which are not disclosed in the prior art.

## Claims

1. A phacoemulsification system (10), comprising:
a phacoemulsification probe (12), comprising:
a piezoelectric crystal (55) configured to vibrate at a mechanical resonance frequency of the crystal;
a needle (16), which is configured to be inserted into a lens capsule (18) of an eye (20) and to be vibrated by the piezoelectric crystal to emulsify a lens of the eye;
an irrigation channel (43a) for receiving irrigation fluid from an irrigation line (43) and flowing the irrigation fluid into the lens capsule; and
an aspiration channel (46a) for removing material from the lens capsule and evacuating the removed material to an aspiration line (46);
an irrigation pump (24) coupled with the irrigation line;
an aspiration pump (26) coupled with the aspiration line;
circuitry (30, 33) configured to measure electrical impedance of the piezoelectric crystal; and
a processor (38), which is configured to receive the measured electrical impedance, and to control an operation of the irrigation pump and the aspiration pump according to the measured electrical impedance.

2. The phacoemulsification system according to claim 1, wherein the processor is configured to control the operation by operating the irrigation pump and the aspiration pump simultaneously.

3. The phacoemulsification system according to claim 1, wherein the processor is configured to control the operation by operating a valve (443, 446) on at least one of the irrigation line and the aspiration line.

4. The phacoemulsification system according to claim 1, wherein the processor is configured to control the operation by operating a valve (443, 446) on at least one of the irrigation channel and the aspiration channel.

## Patentansprüche

1. Phakoemulsifikationssystem (10), umfassend:
eine Phakoemulsifikationssonde (12), umfassend:
einen piezoelektrischen Kristall (55), der konfiguriert ist, um bei einer mechanischen Resonanzfrequenz des Kristalls zu vibrieren;
eine Nadel (16), die konfiguriert ist, um in eine Linsenkapsel (18) eines Auges (20) eingeführt zu werden und durch den piezoelektrischen Kristall in Vibration versetzt zu werden, um eine Linse des Auges zu emulgieren;
einen Spülkanal (43a) zum Aufnehmen von Spülfluid aus einer Spülleitung (43) und zum Einströmen des Spülfluids in die Linsenkapsel; und
einen Aspirationskanal (46a) zum Entfernen von Material aus der Linsenkapsel und zum Ableiten des entfernten Materials zu einer Aspirationsleitung (46);
eine Spülpumpe (24), die mit der Spülleitung gekoppelt ist;
eine Aspirationspumpe (26), die mit der Aspirationsleitung gekoppelt ist;
Schaltlogik (30, 33), die konfiguriert ist, um die elektrische Impedanz des piezoelektrischen Kristalls zu messen; und
einen Prozessor (38), der konfiguriert ist, um die gemessene elektrische Impedanz zu empfangen und einen Betrieb der Spülpumpe und der Aspirationspumpe gemäß der gemessenen elektrischen Impedanz zu steuern.

2. Phakoemulsifikationssystem nach Anspruch 1, wobei der Prozessor konfiguriert ist, um den Betrieb durch gleichzeitiges Betreiben der Spülpumpe und der Aspirationspumpe zu steuern.

3. Phakoemulsifikationssystem nach Anspruch 1, wobei der Prozessor konfiguriert ist, um den Betrieb durch Betätigen eines Ventils (443, 446) an mindestens einer der Spülleitung und der Aspirationsleitung zu steuern.

4. Phakoemulsifikationssystem nach Anspruch 1,
wobei der Prozessor konfiguriert ist, um den Betrieb durch Betätigen eines Ventils (443, 446) an mindestens einem des Spülkanals und des Aspirationskanals zu steuern.

## Revendications

1. Système de phacoémulsification (10), comprenant :
une sonde de phacoémulsification (12), comprenant :
un cristal piézoélectrique (55) conçu pour vibrer à une fréquence de résonance mécanique du cristal ;
une aiguille (16), qui est conçue pour être insérée dans un cristalloïde (18) d'un oeil (20) et pour être mise en vibration par le cristal piézoélectrique afin d'émulsifier un cristallin de l'oeil ;
un canal d'irrigation (43a) pour la réception du liquide d'irrigation à partir d'une ligne d'irrigation (43) et l'écoulement du liquide d'irrigation dans le cristalloïde ; et
un canal d'aspiration (46a) pour le retrait du matériau du cristalloïde et l'évacuation du matériau retiré vers une ligne d'aspiration (46) ;
une pompe d'irrigation (24) accouplée à la ligne d'irrigation ;
une pompe d'aspiration (26) accouplée à la ligne d'aspiration ;
une circuiterie (30, 33) configurée pour mesurer une impédance électrique du cristal piézoélectrique ; et
un processeur (38), qui est configuré pour recevoir l'impédance électrique mesurée et pour commander un fonctionnement de la pompe d'irrigation et de la pompe d'aspiration selon l'impédance électrique mesurée.

2. Système de phacoémulsification selon la revendication 1, dans lequel le processeur est configuré pour commander le fonctionnement en faisant fonctionner la pompe d'irrigation et la pompe d'aspiration simultanément.

3. Système de phacoémulsification selon la revendication 1, dans lequel le processeur est configuré pour commander le fonctionnement en faisant fonctionner une vanne (443, 446) sur l'au moins une parmi la ligne d'irrigation et la ligne d'aspiration.

4. Système de phacoémulsification selon la revendication 1, dans lequel le processeur est configuré pour commander le fonctionnement en faisant fonctionner une vanne (443, 446) sur au moins l'un parmi le canal d'irrigation et le canal d'aspiration.
